## Europäisches Patentamt

## European Patent Office

## Office européen des brevets

(19)

(11) Veröffentlichungsnummer: **0 247 420**
B1

(12) **EUROPÄISCHE PATENTSCHRIFT**

(45) Veröffentlichungstag der Patentschrift:
**25.07.90**

(21) Anmeldenummer: **87106779.9**

(22) Anmeldetag: **11.05.87**

(51) Int. Cl.⁵: **G01N 1/04,** G01N 33/36

(54) Gerät zum Endenordnen von Fasern für eine Faserlängenmessung.

(30) Priorität: **20.05.86 CH 2029/86**

(43) Veröffentlichungstag der Anmeldung:
**02.12.87 Patentblatt 87/49**

(45) Bekanntmachung des Hinweises auf die Patenterteilung:
**25.07.90 Patentblatt 90/30**

(84) Benannte Vertragsstaaten:
**BE DE FR GB IT**

(56) Entgegenhaltungen:
**CH-A- 255 375**
**US-A- 3 057 019**

(73) Patentinhaber: **Siegfried Peyer AG,**
**CH-8832 Wollerau(CH)**

(72) Erfinder: **Völlm, Ernst, Gstaldenstrasse 12,**
**CH-8810 Horgen(CH)**

(74) Vertreter: **Lusuardi, Werther Giovanni, Dr. Lusuardi AG**
**Kreuzbühlstrasse 8, CH-8008 Zürich(CH)**

## Beschreibung

Die Erfindung betrifft ein Gerät zum Endenordnen der Fasern einer Materialprobe für eine Faserlängenmessung, wobei die Materialprobe als Vlies mit parallelisierten Fasern vorliegt und zur Entnahme eines Fasermusters ein Klemmorgan vorgesehen ist, wobei das Klemmorgan zum Klemmen der jeweils in Auszugslage befindlichen Faserenden des Vlieses zum Vlies hin und zum endengeordneten Ausziehen vom Vlies weg bewegbar ist.

Zur Prüfung von Fasern, die eine endliche Länge aufweisen, ist es bekannt, eine Längenmessung der Fasern, die als Stapelmessung bezeichnet ist, durchzuführen. Um bei der Stapelmessung aus einer Materialprobe einen repräsentativen Wert für die ganze Fasermasse zu erhalten, muss für die Probenentnahme besondere Sorgfalt aufgewendet werden.

Es ist zweckmässig, das ganze Gebiet der Längenmessung an textilen Fasern in drei Teilgebiete zu unterteilen, nämlich in

– Faseraufbereitung: darunter versteht man die Herstellung eines parallelisierten Vlieses aus einer oder mehreren Proben aus dem zu prüfenden Material,

– Richten der Fasern; es werden hierzu Fasermusterenden endengeordnet aus dem Vlies ausgezogen, und

– Messung der Faserlänge; hierzu sind Geräte bekannt, die z.B. auf kapazitiver oder optischer Grundlage arbeiten und das Stapeldiagramm, d.h. die Darstellung der Gesamtheit der Fasern, nach der Faserlänge geordnet, selbsttätig ermitteln.

Der Vollständigkeit halber sei erwähnt, dass das erste und zweite Teilgebiet grösstenteils entfällt, wenn ein nicht endengeordnetes Fasermuster (Tuftmuster) hergestellt wird. Der Informationsgehalt eines solchen Musters ist allerdings wesentlich kleiner als derjenige eines endengeordneten Musters.

Es ist ein Gerät gemäss dem Oberbegriff des Patentanspruchs 1 bekannt (CH-A 255 375), welches im wesentlichen aus einem oberen und einem unteren Kammsystem, einer auf zwei Schienen geführten Ausziehzange und einem quer zur Zange beweglichen Umlagerungssystem besteht.

Nachteilig bei diesem bekannten Gerät ist dessen manuelle Bedienung, welche keine Automatisierung der Bewegungsabläufe erlaubt, und die offene Bauweise, welche das Gerät in hohem Masse verschmutzungsanfällig macht und die Ausbildung von Luft-Turbulenzen nicht verhindert.

Hier will die Erfindung Abhilfe schaffen. Die Erfindung, wie sie in den Ansprüchen gekennzeichnet ist, löst die Aufgabe ein Gerät zum Endenordnen der Fasern einer Materialprobe für eine Faserlängenmessung zu schaffen, bei dem eine präzisere Funktion, eine bessere Reproduzierbarkeit, eine geringere Verschmutzungsanfälligkeit, eine bessere Führung der Fasern und insgesamt eine bessere Betriebssicherheit gegenüber den bekannten Geräten erreicht wird.

Die durch die Erfindung erreichten Vorteile sind im wesentlichen darin zu sehen, dass der Bauaufwand des Gerätes verhältnismässig klein, die Steuerungstechnik einfach und die Abmessungen kleingehalten werden können.

Die Erfindung ist in der Zeichnung in einem Ausführungsbeispiel schematisch dargestellt und nachfolgend beschrieben. Es zeigen:

Fig. 1–4 eine schematisch dargestellte Seitenansicht eines Faserrichtgerätes nach dem Stand der Technik, wobei

Fig. 1 das Faserrichtgerät in der Wartestellung,

Fig. 2 das Zustellen des Wagens des Faserrichtgerätes in die Klemmstellung,

Fig. 3 das Ausziehen des Fasermusters aus dem festgehaltenen Vlies und das Rückstellen des Wagens in die Wartestellung und

Fig. 4 die Abgabe des Fasermusters und das Bereitstellen des Faserrichtgerätes für das Ausziehen eines nachfolgenden Fasermusters zeigt, und

Fig. 5 - 8 eine schematisch dargestellte Seitenansicht eines Faserrichtgerätes nach der Erfindung, wobei

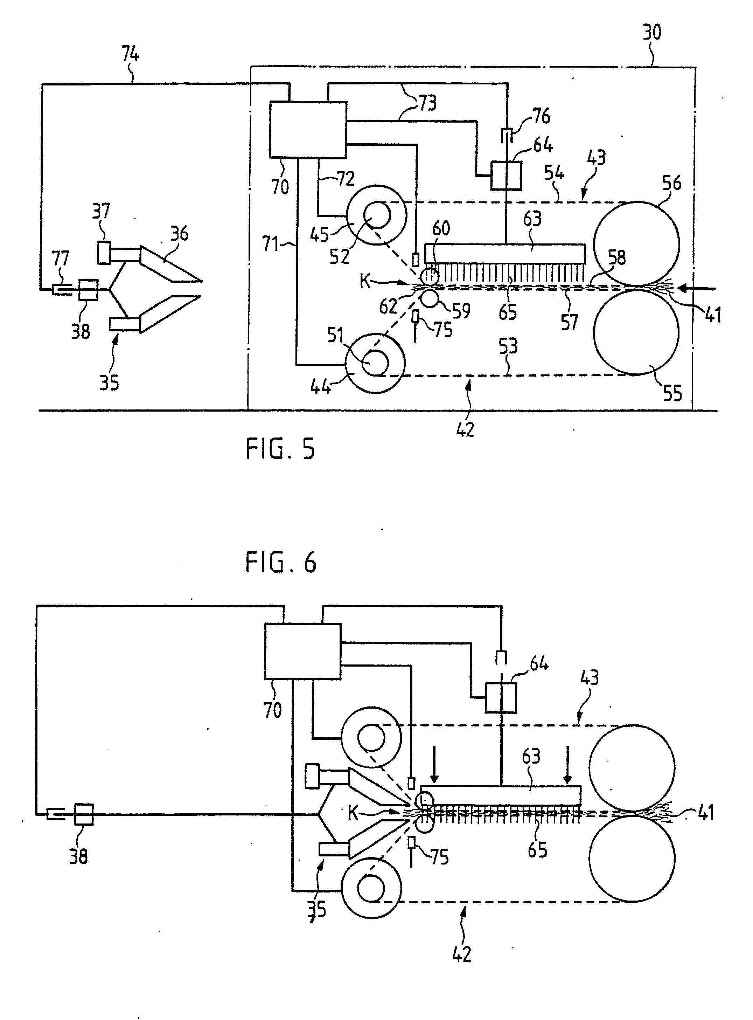

Fig. 5 das Faserrichtgerät in der Wartestellung,

Fig. 6 das Zustellen der Zange in die Klemmstellung

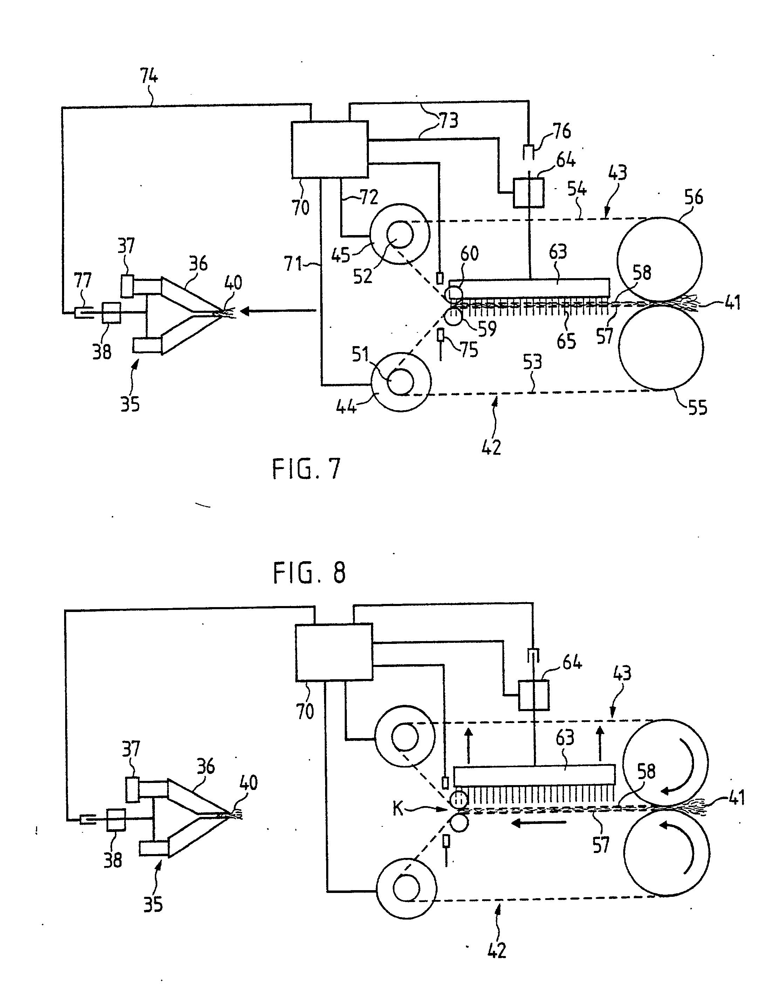

Fig. 7 das Ausziehen des Fasermusters und das Rückstellen der Zange in die Wartestellung und

Fig. 8 das Faserrichtgerät in der Wartestellung beim Vorschieben des freigegebenen Vlieses zeigt.

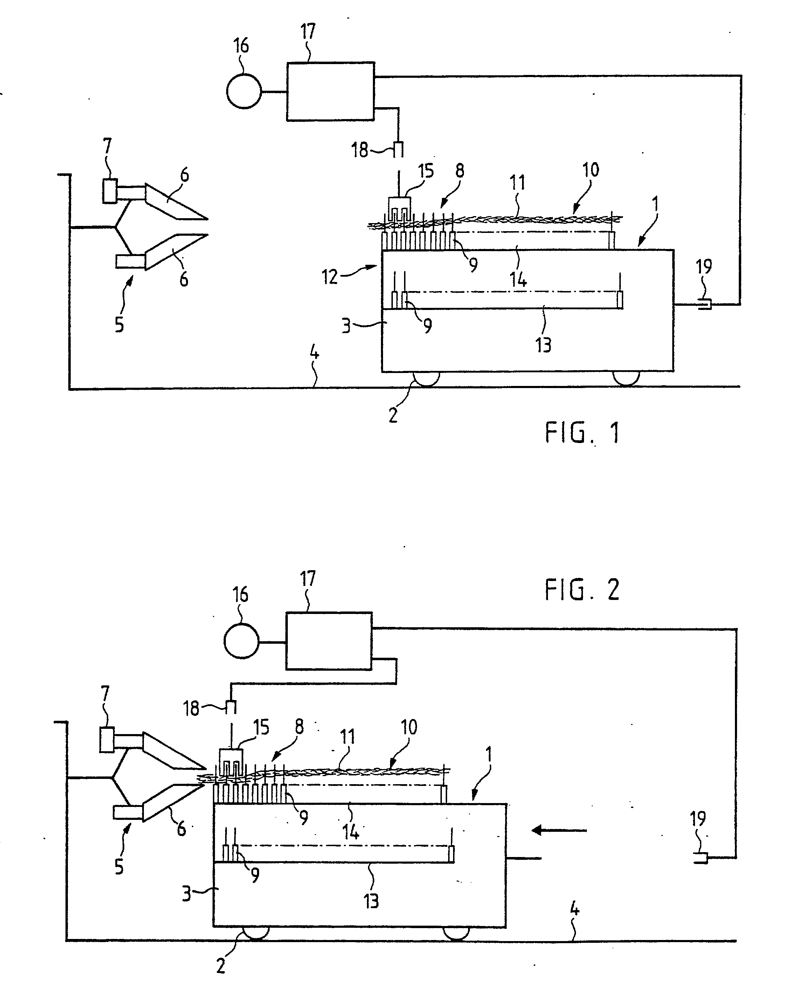

Bei jedem Faserrichtgerät, bei welchem Fasermuster mit endengeordneten Fasern ausgezogen werden, ist es erforderlich, dass die Fadenenden nur soweit vorstehen, dass sie von den hierfür vorgesehenen Klemmmitteln, meistens einer Zange, in einem kleinen Bereich der Faserenden geklemmt werden. Dies wird zwar durch die beiden, in Fig. 1 bis 4 und in Fig. 5 bis 8 beschriebenen Geräten erreicht, jedoch weist das Gerät nach Fig. 1 bis 4 gegenüber dem Gerät nach Fig. 5 bis 8 einige Einschränkungen auf. Die Fasermuster werden bei beiden Geräten aus einem Vlies ausgezogen, das als eine Materialprobe mit parallelisierten Fasern vorliegt. Für die Herstellung eines solchen Vlieses sei auf die beiden Druckschriften der Anmelderin in "Messung der Faserlänge für Kurzstapel-Fasern AL 100/101, Vorschrift und Kurzanleitung für die Mustervorbereitung" verwiesen.

Das Faserrichtgerät nach Fig. 1 bis 4 weist einen Wagen 1, bestehend aus einem Fahrgestell 2 und einem Gehäuse 3, auf, welcher auf einem Fundament 4 hin und her verschiebbar ist. Am Fundament 4 ist weiter ein Klemmorgan 5, z.B. eine Zange, abgestützt. Das Klemmorgan 5 weist zwei Backen 6 auf, von denen mindestens eine Backe durch einen Antrieb 7, z.B. einen Hebelmechanismus, zum Schliessen und Oeffnen des Klemmorgans 5 betätigt werden kann.

Auf dem Wagen 1 ist ein Nadelfeld 8 angeordnet, das aus einzelnen Nadelkämmen 9 zusammengesetzt ist. In das Nadelfeld 8, welches sich aus einer Vielzahl von einzelnen Nadeln zusammensetzt, die reihenweise auf jedem Nadelkamm 9 angeordnet sind, wird eine, auf die vorstehend erwähnte Weise hergestellte Materialprobe 10 manuell eingelegt. Die Materialprobe 10 weist zweckmässig die Form eines Vlieses 11 auf.

Die Nadelkämme 9 sind Teil einer Transportanlage 12, in welcher nach jedem Ausziehen eines Fasermusters der vorderste Nadelkamm 9 in das untere Trum 13 befördert wird, damit neue Faserenden des Vlieses zum Klemmen durch das Klemmorgan 5 bereitstehen. Die im unteren Trum 13 befindlichen Nadelkämme 9 werden an das hintere Ende des oberen Trums 14 befördert und ergänzen das Nadelfeld 18, welches nach dem Ausziehen des Fasermusters den vordersten in das untere Trum 13 geförderten Kamm 9 abgegeben hat.

Damit der Ausziehvorgang immer unter denselben Bedingungen abläuft, ist über dem Nadelfeld ein Eindrücker 15 vorgesehen. Dieser ist im wesentlichen ein Profil mit einer Anzahl Stegen, deren Abstand der Teilung der Nadelkämme 9 im Nadelfeld 8 entspricht. Mit dem Eindrücker 15 wird das Vlies 11 in das Nadelfeld 8 gedrückt. Während des Ausziehens des Fasermusters bleibt der Eindrücker 15 in seiner abgesenkten Lage und hält das Vlies ohne wesentliche Klemmung in seiner Lage.

Für den Betrieb des Faserrichtgerätes nach Fig. 1 dient ein zentraler Antrieb 16 mit einer Steuerung 17. Die Steuerung 17 besteht im wesentlichen aus Schubkurbeltrieben, die über Nockenscheiben gesteuert werden (nicht dargestellt).

Zur Anzeige der jeweiligen Lage der einzelnen Elemente dienen Indikatoren z.B., ein Indikator 18 für die Anzeige der Lage des Eindrückers 15 und ein Indikator 19 für die Anzeige der Stellung des Wagens 1.

Die Funktion des Faserrichtgerätes nach dem Stand der Technik sei anhand von Fig. 2 bis 4 beschrieben, wobei dieselben Bezugszeichen wie in Fig. 1 verwendet werden.

In Fig. 2 ist der Wagen 1 gegen das Klemmorgan 5 vorgeschoben, so dass die Backen 6 die Faserenden des Vlieses 11 klemmen können.

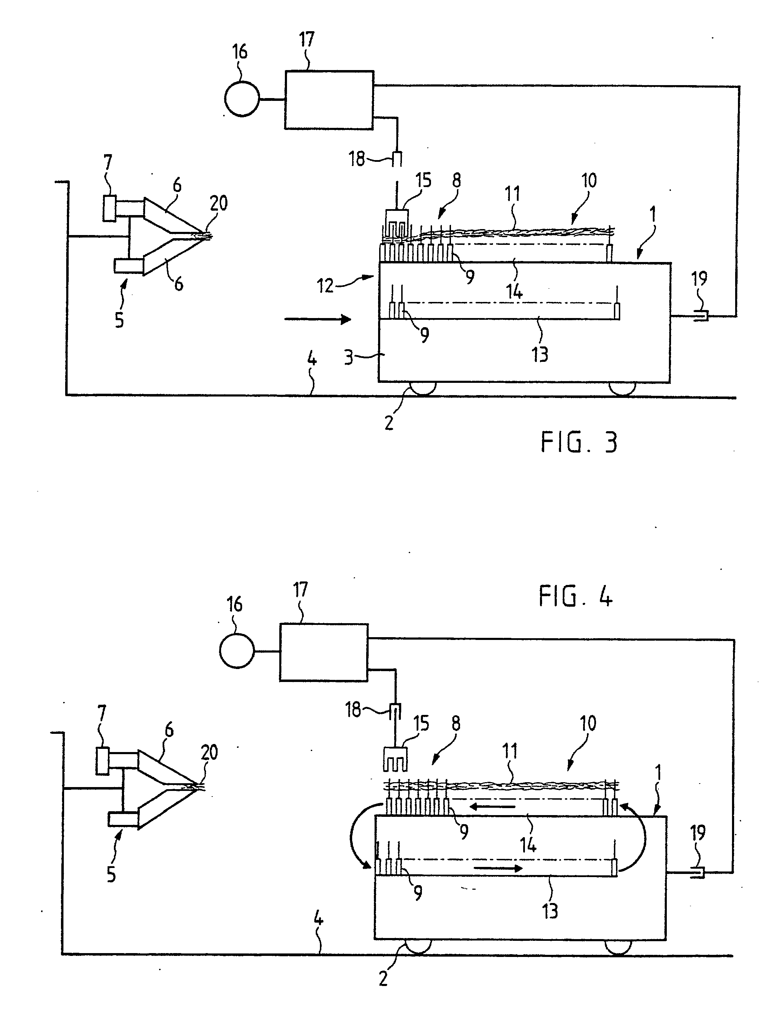

In Fig. 3 ist der Ausziehvorgang beendet. Der Wagen 1 befindet sich wieder in der Wartestellung, während das Klemmorgan 5 ein Fasermuster 20 hält.

In Fig. 4 ist der Eindrücker 15 angehoben und der vorderste Nadelkamm 9 in das untere Trum 13 befördert worden. Nun wird das ganze Nadelfeld 8 um eine Nadelkamm-Teilung vorgeschoben, so dass sich wieder Faserenden in der Auszugsstellung befinden. Das Klemmorgan 5 wird gleichzeitig geöffnet und das Fasermuster daraus entfernt, worauf die nächste Auszugoperation ablaufen kann.

Der ganze Funktionsablauf ist zur besseren Uebersicht in einem Flussdiagramm dargestellt, siehe Tafel 1. Die Zahlen 1 - 4 entsprechen etwa Fig. 1 bis 4, während die Buchstaben a-k die jeweilige Funktion beschreiben.

Die erwähnten Einschränkungen des vorbekannten Faserrichtgerätes bestehen darin, dass wegen des verhältnismässig komplizierten Aufbaues die einzelnen Operationen nicht beliebig schnell durchgeführt werden können. Beim Vor- und Rückschub des Wagens 1 sind Luft-Turbulenzen nicht zu vermeiden, was zu einer Desorientierung der Fasern und damit zu einer Verfälschung der Messungen führen kann. Der Abtransport des ersten Nadelkammes 9 in das untere Trum 13 und das Verschieben des Nadelfeldes 8 sowie das Zurückführen der Nadelkämme an das Nadelfeld 8 sind komplizierte und nicht beliebig schnell durchführbare Operationen, die zudem mit einem gewissen Faserverlust verbunden sein können. Entsprechend dem komplizierten Aufbau des Gerätes - es sind zudem grosse Massen mit langen Hebelgestängen zu bewegen, was die Präzision beeinträchtigt - ist auch der Platzbedarf und Wartungsaufwand entsprechend gross.

Obwohl mit dem erfindungsgemässen Gerät ebenfalls Fasermuster mit endengeordneten Fasern einem Vlies entnommen werden, weicht dieses Gerät wesentlich vom vorbekannten Gerät ab.

In Fig. 5 ist mit der strichpunktierten Linie ein Gehäuse 30 schematisch dargestellt, das den ortsfesten oder stationären Teil des Gerätes bildet. Der bewegliche Teil des Gerätes ist ein Klemmorgan 35, von dem wenigstens die eine Backe 36 mit Hilfe eines Antriebes 37, z.B. eines Exzenters, eines Pneumatik-Zylinders oder eines Hubmagneten, das Oeffnen und Schliessen des Klemmorganes 35 bewirkt wird. Nur das Klemmorgan 35, z.B. in Form einer Zange, ist beweglich ausgebildet und bewegt sich aus der in Fig. 5 ersichtlichen Wartestellung in die Klemmstellung und zurück. Die Bewegung des Klemmorganes 35 braucht jedoch nicht eine lineare Bewegung zu sein. Der Wechsel von der Wartestellung in die Klemmstellung kann auch durch eine Schwenkbewegung oder durch eine Hubbewegung erreicht werden.

In dem Gehäuse 30 sind ein unterer Hülltrieb 42 und ein oberer Hülltrieb 43 angeordnet, deren Antriebe 44, 45 auf der Achse von Antriebsrädern 51, 52 gelagert sind. Die Hüllglieder 53, 54 sind über die Antriebsräder 51, 52 und über Umlenkräder 55, 56 geführt. Wesentlich ist, dass das obere Trum 57 des unteren Hülltriebes 42 und das untere Trum 58 des oberen Hülltriebes 43 nebeneinander liegen und als Führung und Vorschub des zwischen den Umlenkrädern 55, 56 eingeführten Vlieses 41 dienen. Im Bereich der Klemmstelle K, in welche die vorstehenden Faserenden 62 des Vlieses 41 ragen sind für die Führung der Hüllglieder 53, 54 Führungsräder 59, 60 vorgesehen, welche die Anordnung der Antriebsräder 51, 52 der Hülltriebe 42, 43 mit Abstand von der Klemmstelle K ermöglichen.

Ueber den beiden Trum 57, 58 ist eine Kammplatte 63 angeordnet, welche durch einen Hubantrieb 64 anhebbar und absenkbar ist. In der Kammplatte 63 sind quer zur Bewegung des Vlieses 41 angeordnete Nadelreihen 65 befestigt, welche entsprechende Oeffnungen in den Hüllgliedern 53, 54 aufweisen, so dass die Nadeln der Nadelreihen 65 durch oder nur in das Vlies 41 ragen. Auf diese Weise wird das Vlies 41 während des Ausziehens eines Fasermusters gehalten.

Eine Steuerung 70 ermöglicht den selbsttätigen Ablauf des Ausziehens von Fasermustern. Steuerleitungen und Indikatoren ergänzen die Steuerung 70. Die Antriebe 44, 45 sind durch Steuerleitungen 71, 72, der Hubantrieb 64 durch Steuerleitungen 73 und der Antrieb 38 des Klemmorganes 35 durch eine Steuerleitung 74 mit der Steuerung 70 verbunden. Indikatoren 75, 76, 77 melden das Vorhandensein der Faserenden 62, die Stellung des Hubantriebes 64 und die Stellung des Klemmorganes 35.

Das Ausziehen eines Fasermusters aus dem Vlies 41 sei anhand von Fig. 6 bis 8 beschrieben, wobei dieselben Bezugszahlen wie Fig. 5 verwendet werden.

Aus Fig. 6 ist der Vorschub des Klemmorganes 35 an die Klemmstelle K ersichtlich. Der Hubantrieb 64 hat die Kammplatte 63 abgesenkt, so dass die Nadelkämme 65 das Vlies 41 halten. Meldet der Indikator 75 das Vorhandensein von Faserenden 62, schliesst das Klemmorgan 35 die Backen 36.

In Fig. 7 hat das Klemmorgan 35 ein Fasermuster 40 aus dem Vlies 41 endgeordnet ausgezogen. In Fig. 8 ist das Ende der Auszugoperation dargestellt. Das Klemmorgan 35 öffnet zum Entfernen des ausgezogenen Fasermusters 40 die Backen 36, die Kammplatte 63 wird durch den Hubantrieb 64 aus dem Bereich der Trum 57, 58 der Hülltriebe 42, 43 entfernt und die Hülltriebe 42, 43 schieben das Vlies 41 soweit vor, dass neue Faserenden 62 in die Klemmstelle K ragen. Ein entsprechendes Flussdiagramm des Funktionsablaufes zeigt Tafel 2, wobei die Zahlen 5 - 8 etwa der Darstellung in den Fig. 5 bis 8 entsprechen. Die Buchstaben I - w beschreiben die jeweiligen Funktionen.

Die Hüllglieder 53, 54 der Hülltriebe 42, 43 können in verschiedener Weise ausgeführt sein. Wesentlich ist, dass sie Querschlitze aufweisen, deren Abstand der Teilung der Nadelreihen 65 der Kammplatten 63 entspricht. In der einen Ausführungsform werden zwei mit Abstand nebeneinanderliegende Riemen verwendet, wobei Querstäbe mit ihren Enden in den Riemen befestigt sind. Die lichte Weite zwischen den Riemen ist etwas grösser als die Breite der Nadelreihen 65 der Kammplatte 63. Bei einer andern Ausführungsform besteht das Hüllglied aus zwei mit Abstand angeordneten Gliederketten, bei denen die Querstäbe die Gelenkachsen der Kettenglieder bilden. Es könnte jedoch auch ein Flachband verwendet werden, in welchem die den Nadelreihen 65 entsprechenden Schlitze ausgespart sind.

Der Hubantrieb 64 kann als elektromechanischer, hydraulischer, pneumatischer oder elektromagnetischer Antrieb ausgebildet sein. Das gleiche gilt auch für den Antrieb 38 des Klemmorganes 35. Die Bewegung des Klemmorgans 35 zur Klemmstelle K kann als Linearbewegung, Schwenkbewegung oder Hubbewegung gebildet werden. Die Antriebe 44, 45 der Hülltriebe 42, 43 sind beispielsweise Schrittmotoren, mit denen der gewünschte Vorschub exakt eingehalten werden kann.

Die Antriebe der Hülltriebe 42, 43 können auch in anderer Weise angetrieben sein, z.B. durch einen pneumatischen oder elektrischen Klinkenantrieb.

Anstelle von zwei Hülltrieben 42, 43 könnte auch nur der untere Hülltrieb 42 für den Vorschub des Vlieses 41 verwendet werden. In diesem Fall können zusätzliche Mittel zum Festhalten des Vlieses auf dem Hüllglied 53 des unteren Hülltriebes verwendet werden, z.B. durch Absaugen, durch elektrostatische Aufladung o.dgl.

Die Vorteile des beschriebenen erfindungsgemässen Faserrichtgerätes bestehen in einer mechanisch einfacheren Lösung, einer einfacheren Steuerungstechnik, kleineren Abmessungen, geringeren Luft-Turbulenzen, präzisen Funktionen, geringeren Verschmutzungsanfälligkeit, exakter Führung der Fasern, kleinerem Faserverlust und besserer Wartungsmöglichkeit.

Von besonderer Bedeutung ist die bessere Automatisierbarkeit, da die ganze Steuerung als Folgesteuerung ausgelegt und auch die Zuführung des Vlieses 41 automatisiert werden kann.

Im weitern sei noch erwähnt, dass der Abstand der Nadeln der Nadelkämme 65 variiert werden kann, da die Nadelkämme ihre Lage in der Klemmplatte 63 nicht wechseln. Auch müssen keine einzelnen Nadelkämme 65 bewegt werden, so dass der Abstand der Nadelkämme sehr klein gewählt und dementsprechend auch der Klemmbereich der Faserenden 62 kleiner gehalten werden kann.

Legende zu Tabelle I und II

**Tabelle I**

M  Musterherstellung

a  Wagen 1 in Wartestellung?

b  Wagen 1 in Wartestellung bringen

c  Eindrücker 15 unten?

d  Eindrücker 15 nach unten bringen

e  Wagen 1 zum Klemmorgan 5 bringen

f  Klemmorgan 15 schliessen

g  Wagen-Rückzugbewegung (Ausziehen)

h  Klemmorgan 5 öffnen

i  Fasermuster 20 entfernen

j  Eindrücker 15 nach oben bringen

k  Kamm-/Materialvorschub vorderster Kamm 9 fällt auf unteres Trum 13

**Tabelle II**

M  Musterherstellung

l  Klemmorgan 35 in Wartestellung?

m  Klemmorgan 35 in Wartestellung bringen

n  Kammplatte 63 oben?

o  Kammplatte 63 nach oben bringen

p  Faserenden 62 vorstehend?

q  Vliestransport um Kammteilung

r  Kammplatte 63 nach unten bringen (Einstechen)

s  Klemmorgan 35 an Klemmstelle K bringen

t  Klemmorgan 35 schliessen

u  Klemmorgan-Rückzugbewegung

v  Klemmorgan 63 öffnen

w  Fasermuster 40 entfernen

## Patentansprüche

1. Gerät zum Endenordnen der Fasern einer Materialprobe für eine Faserlängenmessung, wobei die Materialprobe als Vlies (41) mit parallelisierten Fasern vorliegt und zur Entnahme eines Fasermusters ein Klemmorgan (35) vorgesehen ist, wobei das Klemmorgan (35) zum Klemmen der jeweils in Auszugslage befindlichen Faserenden des Vlieses (41) zum Vlies (41) hin und zum endengeordneten Ausziehen vom Vlies (41) weg bewegbar ist, dadurch gekennzeichnet, dass ein stationäres Gehäuse (30) vorgesehen ist, in welchem das Vlies (41) durch eine Transportanlage, welche zwei zusammenwirkende Hülltriebe (42, 43) mit Hüllgliedern (53, 54) und dazugehörigen Antrieben (44, 45) umfasst, in die Fasermuster-Entnahmestellung vorschiebbar ist, wobei das Vlies (41) während des Ausziehens des Fasermusters (40) durch Nadelkämme (65) festgehalten ist, welche in einer Kammplatte (63) aufgenommen sind, deren Hubbewegung mit der Vorschubbewegung der Transportanlage koordiniert ist und die Hülltriebe (42, 43) der Transportanlage zwei Hüllglieder (53, 54) mit je einem Trum (57, 58) aufweisen, welche das Vlies (41) auf der Unter- und Oberseite für dessen Vorschub und Führung umfassen, wobei mindestens eines der Trum (57; 58), vorzugsweise das Trum (58) des oberseitigen Hülltriebes (43), mit Öffnungen für den Durchgang der Nadeln der Nadelkämme (65) der Kammplatte (63) beim Festhalten des Vlieses (4i) während des Ausziehens des Fasermusters (40) versehen ist.

2. Gerät nach Anspruch 1, dadurch gekennzeichnet, dass die zwei Hüllglieder (53, 54) der Transportanlage aus je einem Riemen, beispielsweise einem Riemen mit Befestigungsöffnungen zusammengesetzt ist, zwischen dem senkrecht zur Bewegungsrichtung der Hüllglieder. (53, 54) Querstäbe unter Bildung von Querschlitzen angeordnet sind.

3. Gerät nach Anspruch 1, dadurch gekennzeichnet, dass die Transportbahn, bzw. die Transportbahnen als Raupe mit zwei, aus Kettengliedern zusammengesetzten Randketten ausgebildet ist, wobei die Gelenkbolzen der Gelenke der Kettenglieder durch die Querstäbe gebildet sind.

4. Gerät nach einem der Ansprüche 1 bis 3, dadurch gekennzeichnet, dass das Klemmorgan (35) mit einem Antrieb (38) für eine Linear-, Schwenk- oder Hubbewegung zum Vlies (41) hin für das Klemmen der Faserenden (62) und vom Vlies (41) weg zum Ausziehen des Fasermusters (40) ausgerüstet ist.

5. Gerät nach einem der Ansprüche 1 bis 4, dadurch gekennzeichnet, dass die zwei Hüllglieder (53, 54) der Transportanlage aus in stirnseitigen Kanälen geführte, durch Distanzglieder mit Abstand gehaltene Querstäbe zusammengesetzt sind, wobei der Antrieb über ein gezähntes Rad erfolgt, dessen Zähne zwischen die Querstäbe eingreifen.

6. Gerät nach einem der Ansprüche 1 bis 5, dadurch gekennzeichnet, dass der Abstand der Nadeln der Nadelkämme (65) unterschiedlich gross ist.

7. Gerät nach einem der Ansprüche 1 bis 5, dadurch gekennzeichnet, dass die zwei zusammenwirkenden Hülltriebe (42, 43) mit Hüllgliedern (53, 54) und dazugehörigen Antrieben (44, 45) das Vlies (41) synchron bewegen, wobei je eines der Hülltriebe (42; 43) entweder auf der Ober- und auf der Unterseite oder auf den beiden Seitenflächen des Vlieses (41) angelegt sind.

## Revendications

1. Dispositif d'ordonnancement d'extrémités de fibres d'une éprouvette de matière en vue de permettre la mesure de la longueur des fibres, conformément auquel l'éprouvette de matière se présente sous forme d'un voile, matelas ou d'une nappe à fibres (41) disposées en parallèle et un organe de serrage (35) est prévu pour le prélèvement d'un échantillon de fibres, où l'organe de serrage (35) est déplaçable en direction de la nappe (41) en vue du serrage des extrémités des fibres de la nappe (41) se trouvant en position d'extraction et à partir de la nappe (41) en vue de l'extraction ordonnancée des extrémités, caractérisé en ce que l'on a prévu un boîtier stationnaire ou immobile (30) dans lequel on peut faire avancer ou progresser la nappe (41) jusque dans la position de prélèvement d'échantillons de fibres à l'aide d'une installation de transport, qui englobe deux entraînements à enveloppement coopérants (42, 43) avec des éléments d'enveloppement (53, 54) et des dispositifs d'entraînement ou d'actionnement leur appartenant (44, 45), où on maintient fermement la nappe (41), au cours de l'extraction de l'échantillon de fibres (40), à l'aide de peignes à aiguilles (65) qui sont logés dans une plaque à peignes (63) dont le mouvement d'élévation ou ascendant est coordonné avec le mouvement d'avancement de l'installation de transport et les entraînements à enveloppement (42, 43) de l'installation de transport présentent deux éléments d'enveloppement (53, 54) avec chacun une galerie (57, 58), qui enveloppent la nappe (41) du côté inférieur et du côté supérieur pour l'avancement ou la progression de cette nappe et sa conduite ou guidage, où au moins l'une des galeries (57, 58), de préférence la galerie (58) de l'entraînement à enveloppement supérieur (43) est pourvue d'ouvertures pour le passage des aiguilles des peignes à aiguilles (65) de la plaque à peignes (63) au cours de la retenue ou assujettissement de la nappe (41 ) pendant l'extraction de l'échantillon de fibres (40).

2. Appareil suivant la revendication 1, caractérisé en ce que les deux éléments d'enveloppement (53, 54) de l'installation de transport sont chacun constitués d'une courroie, par exemple une courroie avec des ouvertures d'assujettissement, entre lesquelles des barreaux transversaux sont agencés perpendiculairement à la direction de déplacement des éléments d'enveloppement (53, 54) avec formation de fentes transversales.

3. Appareil suivant la revendication 1, caractérisé en ce que la bande de transport, ou les bandes de transport, sont conçues sous forme de chenille avec deux chaînes à rebord composées de maillons de chaîne, où les pivots des articulations des maillons de chaîne sont formés par les barreaux transversaux.

4. Appareil suivant l'une des revendications 1 à 3, caractérisé en ce que l'organe de serrage (35) est équipé d'un dispositif d'entraînement ou d'actionnement (38) destiné à lui imprimer un mouvement linéaire, oscillant ou de levage ou d'élévation pour faire avancer l'organe de serrage (35) vers la nappe (41) en vue du serrage des extrémités des fibres (62) et pour écarter l'organe de serrage (35) de la nappe (41) en vue de l'extraction de l'échantillon de fibres (40).

5. Appareil suivant l'une des revendications 1 à 4, caractérisé en ce que les deux éléments d'enveloppement (53, 54) de l'installation de transport se composent de barreaux transversaux maintenus à distance par des éléments d'écartement, conduits ou guidés dans des canaux frontaux, où l'entraînement s'effectue par l'intermédiaire d'une roue dentée dont les dents prennent entre les barreaux transversaux.

6. Appareil suivant l'une des revendications 1 à 5, caractérisé en ce que a distance des aiguilles des peignes à aiguilles (65) varie diversement.

7. Appareil suivant l'une des revendications 1 à 5, caractérisé en ce que les deux entraînements à enveloppement coopérants (42, 43) avec les éléments d'enveloppement (53, 54) et les dispositifs d'entraînement ou d'actionnement leur appartenant (44, 45) déplacent la nappe (41) de façon synchrone, où un des entraînements à enveloppement (42, 43) se trouve disposé soit sur la surface supérieure et sur la surface inférieure, ou sur les deux surfaces latérales de la nappe (41).

**Claims**

1. Apparatus for aligning and extracting fibers from the end of a sample of material for fiber length measurement, whereby the sample of material is present in the form of a sliver (41) with parallelized fibers and a clamping member (35) for extracting a fiber sample is provided, whereby the clamping member (35) is movable towards the sliver (41) for clamping those fiber ends of the sliver (41) which are in an extracting position and away from the sliver (41) for extracting the fiber ends, characterized in that a stationary casing (30) is provided, in which the sliver (41) is advanceable by means of transporting means comprising two cooperating conveyors (42, 43) with conveyor members (53, 54) and corresponding drives (44, 45) towards the extracting position of the fiber sample, whereby the sliver (41) is retained during extraction of the fibre sample (40) by needle combs (65) held in a needle comb plate (63), the stroke movement of which is coordinated with the advancing movement of the transporting means and that the conveyors (42, 43) of the transporting means are provided with two conveyor members (53, 54) having each a strand (57, 58), which grasps the sliver (41) on its lower and upper side permitting its advancing and guiding, whereby at least one of the strands (57; 58), preferably the strand (58) of the upper conveyor (43), is provided with openings for permitting the passage of the needles of the needle combs (65) of the needle comb plate (63) while the sliver (41) is clamped during the extraction of the fibre sample (40).

2. Apparatus according to claim 1, characterized in that the two conveyor members (53, 54) of the transporting means are composed each of a belt, for example a belt with fixation openings, between which transverse rods are arranged vertically to the moving direction of the conveyor members (53, 54), thereby defining transverse slots.

3. Apparatus according to claim 1, characterized in that the transporting means comprise a caterpillar with two rim chains composed of chain links, whereby the hinge bolts of the hinges of the chain links are formed by the transverse rods.

4. Apparatus according to one of the claims 1 to 3, characterized in that the clamping member (35) is provided with a drive (38) for a linear, pivoting or lifting movement towards the sliver (41) for the clamping of the fiber ends (62) and away from the sliver (41) for the extraction of the fiber sample (40).

5. Apparatus according to one of the claims 1 to 4, characterized in that the two conveyor members (53, 54) of the transporting means are composed of transverse rods guided in frontal channels, spaced apart by distance members, whereby the drive is effected by means of a toothed wheel, the teeth of which engage with the transverse rods.

6. Apparatus according to one of the claims 1 to 5, characterized in that the distance between the needles of the needle combs (65) is variable.

7. Apparatus according to one of the claims 1 to 5, characterized in that the two cooperating conveyors (42, 43) with the corresponding conveyor members (53, 54) and drives (44, 45) are arranged to move the sliver (41) synchronously, whereby the two conveyors (42; 43) are put against either the upper and lower side each or against the two lateral sides of the sliver (41).

FIG. 1

FIG. 2

FIG. 3

FIG. 4

FIG. 5

FIG. 6

FIG. 7

FIG. 8